Europäisches Patentamt

European Patent Office

Office européen des brevets

(11) Veröffentlichungsnummer: **0 088 998**
**B1**

(12) # EUROPÄISCHE PATENTSCHRIFT

(45) Veröffentlichungstag der Patentschrift:
27.11.85

(21) Anmeldenummer: 83102292.6

(22) Anmeldetag: 09.03.83

(51) Int. Cl.⁴: **C 07 B 55/00**, C 07 C 149/247

(54) Verfahren zur Racemisierung von optisch aktivem S-(Carboxymethyl)-cystein.

(30) Priorität: 13.03.82 DE 3209232

(43) Veröffentlichungstag der Anmeldung:
21.09.83 Patentblatt 83/38

(45) Bekanntmachung des Hinweises auf die Patenterteilung:
27.11.85 Patentblatt 85/48

(84) Benannte Vertragsstaaten:
BE CH DE FR GB LI NL SE

(56) Entgegenhaltungen:
EP - A - 0 057 092
DE - A - 2 309 180
DE - A - 2 740 380
DE - B - 1 543 919

Chemical Abstracts Band 88, Nr. 25, 19. Juni 1978,
Columbus, Ohio, USA M.G. RYZHOV et al. "Acidic amino
acid racemates", Seite 829, rechte Spalte, Abstract Nr.
191477K
Chemical Abstracts Band 72, Nr. 7, 16. Februar 1970,
Columbus, Ohio, USA M. ANDO et al. "Catalytic
activities of salicylaldehyde derivatives. I. Catalytic
effects of 4-formyl-3-hydroxyphenyltrimethyl ammonium
bromide on the racemization of L-amino acids", Seite
389, rechte Spalte, Abstract Nr. 32194u

(73) Patentinhaber: Degussa Aktiengesellschaft,
Weissfrauenstrasse 9, D-6000 Frankfurt am Main 1 (DE)

(72) Erfinder: Bethge, Horst, Schwalbenstrasse 2A,
D-6450 Hanau 1 (DE)
Erfinder: Kleemann, Axel, Dr., Dipl.-Chem.,
Greifenhagenstrasse 25, D-6450 Hanau 9 (DE)
Erfinder: Martens, Jürgen, Dr., Dipl.-Chem.,
Hochstrasse 5, D-8755 Alzenau (DE)

## Beschreibung

Die Erfindung betrifft ein Verfahren zur Racemisierung von optisch aktivem S-(Carboxymethyl)-cystein. Bei der Gewinnung des für pharmazeutische Zwecke benötigten S-(Carboxymethyl)-(R)-cysteins durch Trennung des Racemats S-(Carboxymethyl)-(R S)-cystein fällt zwangsläufig als Nebenprodukt das S-(Carboxymethyl)-(S)-cystein an. Durch die Racemisierung wird dieses zur Gewinnung weiteren S-(Carboxymethyl)-(R)-cysteins nutzbar.

Ein Verfahren zur Racemisierung von optisch aktivem S-(Carboxymethyl)-cystein ist bisher nicht beschrieben. Für die Racemisierung der optisch aktiven Formen anderer Schwefel enthaltender Aminosäure, wie Methionin oder Homocystein, werden diese in vorzugsweise neutralem wässrigem Medium bei Temperaturen über 150°C behandelt (US-A-3213106). Optische Isomere der N-Acylderivate der Aminosäure werden als Salze in wässriger Lösung bei pH-Werten von 2 bis 11 mit Hilfe von Ketenen racemisiert (DE-A-2740380). Die bekannten Verfahren sind im Falle des S-(Carboxymethyl)-cysteins ungeeignet. Sofern überhaupt eine Racemisierung eintritt, sind die Ausbeuten unbefriedigend.

Es ist nun ein Verfahren für die Racemisierung von optisch aktivem S-(Carboxymethyl)-cystein gefunden worden, das dadurch gekennzeichnet ist, dass das optisch aktive S-(Carboxymethyl)-cystein bei erhöhter Temperatur in einem sauren wässrigen Medium behandelt wird, das einen aromatischen Aldehyd enthält, der in ortho-Stellung zur Aldehyd-Gruppe eine Hydroxy-Gruppe aufweist. Dieses Verfahren eignet sich hervorragend zur Racemisierung von optisch aktivem S-(Carboxymethyl)-cystein und ergibt hohe Ausbeuten an S-(Carboxymethyl)-(R S)-cystein. Dieses fällt in so reiner Form an, dass es unmittelbar als Ausgangssubstanz zur Trennung des Racemats zwecks Gewinnung des gewünschten optischen Isomeren eingesetzt werden kann. Nach dem erfindungsgemässen Verfahren kann sowohl S-(Carboxymethyl)-(R)-cystein als auch S-(Carboxymethyl)-(S)-cystein racemisiert werden.

Für die Durchführung des erfindungsgemässen Verfahrens wird das zu racemisierende optisch aktive S-(Carboxymethyl)-cystein in ein saures wässriges, den Aldehyd enthaltendes Medium eingesetzt. Die Mengenverhältnisse sind weitgehend frei wählbar. Zweckmässig ist es jedoch, nur so viel des S-(Carboxymethyl)-cysteins zu nehmen, dass sich Lösungen ergeben, und zwar Lösungen die vorzugsweise etwa 5 bis 40, insbesondere 5 bis 25, Gewichts-% des S-(Carboxymethyl)-cysteins enthalten. Die Säurekonzentration wird im allgemeinen so gewählt, dass der pH-Wert des Mediums kleiner als 6, vorzugsweise kleiner als 3 und insbesondere 1 oder kleiner als 1 ist. Das Medium enthält zweckmässigerweise je Mol des S-(Carboxymethyl)-cysteins mindestens 0,01 Mol, vorzugsweise 0,01 bis 10 Mol, insbesondere 0,1 bis 1,0 Mol, des Aldehyds.

Zur Einstellung des pH-Wertes dienen starke Mineralsäuren, wie Schwefelsäure, Phosphorsäure, Bromwasserstoffsäure oder insbesondere Chlorwasserstoffsäure.

Die Racemisierung erfolgt in Gegenwart aromatischer Aldehyde, die in ortho-Stellung zur Aldehyd-Gruppe eine Hydroxy-Gruppe aufweisen. Mit Vorteil wird als Aldehyd ein Benzaldehyd oder ein Naphthaldehyd verwendet, der in ortho-Stellung zur Aldehyd-Gruppe eine Hydroxy-Gruppe hat und im übrigen durch eine oder mehrere Alkyl-Gruppen, bevorzugt durch eine Alkyl-Gruppe mit einem Kohlenstoffatom oder zwei Kohlenstoffatomen, bevorzugt mit einem Kohlenstoffatom, substituiert sein kann, insbesondere aber nicht substituiert ist. Geeignete Aldehyde sind beispielsweise 2-hydroxy-3-methylbenzaldehyd, 2-Hydroxy-4-methylbenzaldehyd, 2-Hydroxy-5-methylbenzaldehyd, 2-Hydroxy-6-methylbenzaldehyd, 2-Hydroxy-4,5-dimethylbenzaldehyd, 2-Hydroxy-1-naphthaldehyd, 2-Hydroxy-3-äthyl-benzaldehyd, 2-Hydroxy-4-äthylbenzaldehyd, 2-Hydroxy-5-äthylbenzaldehyd und insbesondere Salicylaldehyd.

Zur Racemisierung wird das optisch aktive S-(Carboxymethyl)-cystein in dem Medium bei erhöhten Temperaturen behandelt, zweckmässigerweise bei Temperaturen etwa zwischen 80 und 150°C, vorzugsweise bei Temperaturen zwischen 90 und 130°C, insbesondere bei Temperaturen um den Siedepunkt des Mediums. Wenngleich beliebige Drücke in Frage kommen, ist es im allgemeinen zweckmässig, vom Normaldruck nicht wesentlich abzuweichen. Gegebenenfalls ist es wegen des Vorliegens flüchtiger Substanzen erforderlich, einen der Temperatur entsprechend erhöhten Druck zu wählen.

*Beispiel 1:*

545 g S-(Carboxymethyl)-(S)-cystein und 35 g Salicylaldehyd wurden in einer Mischung von 250 ml konzentrierter Schwefelsäure und 3000 ml Wasser gelöst. Die Lösung wurde während 12 h unter Rückfluss auf Siedetemperatur gehalten, dann mit 25 g Aktivkohle versetzt und filtriert. Aus dem Filtrat wurden 250 ml Flüssigkeit abgedampft. Der Rest wurde mittels Natriumhydroxid auf den pH-Wert 3,0 eingestellt und dann bei 10°C unter Absaugen filtriert. Der Filterrückstand wurde so lange mit kaltem Wasser gewaschen, bis er frei von Sulfationen war, dann mit 100 ml Methanol nachgewaschen und bei 105°C und 25 mbar getrocknet. Das so gewonnene Produkt war S-(Carboxymethyl)-(R S)-cystein. Es war 99,5%ig und optisch inaktiv. Die Ausbeute betrug 458 g, entsprechend 84%, bezogen auf das eingesetzte S-(Carboxymethyl)-(S)-cystein.

*Beispiel 2:*

Eine Mischung von 36 g S-(Carboxymethyl)-(S)-cystein, 2 ml Salicylaldehyd, 30 g konzentrierter Schwefelsäure und 300 ml Wasser wurde während 20 h unter Rückfluss auf Siedetemperatur gehalten, dann mit 2 g Aktivkohle versetzt und filtriert. Das Filtrat wurde mit Natriumhydroxid auf den pH-Wert 2,5 eingestellt und dann filtriert. Der Filterrückstand wurde mit 20 ml kaltem Wasser gewaschen und bei 80°C und 25 mbar getrocknet. Es wurden 27 g S-(Carboxymethyl)-(R S)-cystein gewonnen, entsprechend einer Ausbeute von 75%, bezogen auf das eingesetzte S-(Carboxymethyl)-(S)-cystein. Das Produkt war optisch inaktiv. Die Elementaranalyse ergab: C=33,70% (33,51%); H=5,20% (5,06%); N=7,80% (7,82%); S=17,95% (17,89%) — (in Klammern die für $C_5H_9NO_4S$ berechneten Werte).

*Beispiel 3:*

Es wurde wie nach Beispiel 2 verfahren, jedoch wurden 72 g S-(Carboxymethyl)-(S)-cystein und statt der Schwefelsäure 300 ml konzentrierte wässrige Chlorwasserstoffsäure eingesetzt. Die Ausbeute an S-(Carboxymethyl)-(R S)-cystein betrug 60 g, entsprechend 83%. Das Produkt war optisch inaktiv.

*Beispiel 4:*

Eine Mischung von 36 g S-(Carboxymethyl)-(S)-cystein, 2 ml Salicylaldehyd, 46 ml konzentrierter Chlorwasserstoffsäure und 300 ml Wasser wurde während 7 h unter Rückfluss auf Siedetemperatur gehalten und dann mit Natriumhydroxid auf den pH-Wert 3,0 eingestellt. Das hierbei abgeschiedene S-(Carboxymethyl)-(R S)-cystein wurde abfiltriert, mit 50 ml kaltem Wasser gewaschen und bei 80°C und 20 mbar getrocknet. Die Ausbeute betrug 31 g, entsprechend 86%. Das Produkt zeigte geringe optische Aktivität zufolge eines Gehalts von 3% nicht racemisiertem S-(Carboxymethyl)-cystein.

*Beispiel 5:*

Es wurde wie nach Beispiel 4 verfahren, jedoch wurde die Mischung während 5 h in einem Autoklaven auf 140°C gehalten. Die Ausbeute betrug 32 g, entsprechend 89%. Das Produkt war optisch inaktiv.

*Beispiel 6:*

Es wurde wie nach Beispiel 4 verfahren, jedoch wurden 36 g S-(Carboxymethyl)-(R)-cystein eingesetzt. Die Ausbeute betrug 30 g, entsprechend 83%. Das Produkt enthielt 3% nicht racemisiertes S-(Carboxymethyl)-(R)-cystein.

*Beispiel 7:*

Es wurde wie nach Beispiel 2 verfahren, jedoch wurden 72 g S-(Carboxymethyl)-(S)-cystein, nur 1 ml Salicylaldehyd und als Säure 300 ml konzentrierte Chlorwasserstoffsäure eingesetzt. Die Ausbeute betrug 60 g, entsprechend 83%. Das Produkt war optisch inaktiv.

*Beispiel 8:*

Es wurde wie nach Beispiel 2 verfahren, jedoch wurden als Aldehyd 2 g 2-Hydroxy-4-methylbenzaldehyd eingesetzt. Die Ausbeute betrug 26 g, entsprechend 72%. Das Produkt war optisch inaktiv.

*Beispiel 9:*

Es wurde wie nach Beispiel 2 verfahren, jedoch wurden als Aldehyd 3 g 2-Hydroxy-5-methylbenzaldehyd eingesetzt. Die Ausbeute betrug 25 g, entsprechend 69%. Das Produkt war optisch inaktiv.

*Beispiel 10:*

Es wurde wie nach Beispiel 1 verfahren, jedoch wurden als Säure 150 ml Phosphorsäure eingesetzt. Die Ausbeute betrug 440 g, entsprechend 81%. Das Produkt war optisch inaktiv.

*Beispiel 11:*

36 g S-(Carboxymethyl)-cystein, das zu 80% aus S-(Carboxymethyl)-(S)-cystein und zu 20% aus S-(Carboxymethyl)-(R)-cystein bestand, wurden mit 2 ml Salicylaldehyd, 16 ml konzentrierter Chlorwasserstoffsäure und 300 ml Wasser vermischt. Die Mischung wurde während 8 h unter Rückfluss auf Siedetemperatur gehalten und dann mit Natriumhydroxid auf den pH-Wert 3,0 eingestellt. Das hierbei abgeschiedene S-(Carboxymethyl)-(R S)-cystein wurde abfiltriert, mit 50 ml kaltem Wasser gewaschen und bei 80°C und 25 mbar getrocknet. Die Ausbeute betrug 31 g, entsprechend 86%. Das Produkt war optisch inaktiv.

*Beispiel 12:*

Eine Mischung von 36 g S-(Carboxymethyl)-(S)-cystein, 350 ml Wasser, 30 g konzentrierter Schwefelsäure und 3 g 2-hydroxy-1-naphthaldehyd wurde während 24 h unter Rückfluss auf Siedetemperatur gehalten, dann mit 12 g Aktivkohle versetzt und filtriert. Das Filtrat wurde mit Natriumhyderoxid auf den pH-Wert 2,5 eingestellt. Das hierbei abgeschiedene S-(Carboxymethyl)-(R S)-cystein wurde abfiltriert, mit 20 ml 0,1n Schwefelsäure und dann mit 20 ml Wasser gewaschen und schliesslich bei 80°C und 25 mbar getrocknet. Die Ausbeute betrug 27 g, entsprechend 75%. Das Produkt zeigte geringe optische Aktivität zufolge eines Gehalts von 3% nicht racemisiertem S-(Carboxymethyl)-(S)-cystein.

## Patentansprüche

1. Verfahren zur Racemisierung von optisch aktivem S-(Carboxymethyl)-cystein, dadurch gekennzeichnet, dass das optisch aktive S-(Carboxymethyl)-cystein bei erhöhter Temperatur in einem sauren wässerigen Medium behandelt wird, das einen aromatischen Aldehyd enthält, der in ortho-Stellung zur Aldehyd-Gruppe eine Hydroxy-Gruppe aufweist.

2. Verfahren nach Anspruch 1, dadurch gekennzeichnet, dass das Medium Salicylaldehyd enthält.

3. Verfahren nach Anspruch 1 oder 2, dadurch gekennzeichnet, dass das Medium mindestens 0,01 Mol des Aldehyds je mol des optisch aktiven S-(Carboxymethyl)-cysteins enthält.

4. Verfahren nach einem oder mehreren der Ansprüche 1 bis 3, dadurch gekennzeichnet, dass das Medium einen pH-Wert von höchstens 1 hat.

5. Verfahren nach einem oder mehreren der Ansprüche 1 bis 4, dadurch gekennzeichnet, dass die Behandlung bei Temperaturen zwischen 90 und 130°C erfolgt.

## Claims

1. A process for racemising optically-active S-(carboxymethyl)-cysteine, characterised in that the optically-active S-(carboxymethyl)-cysteine is treated at an elevated temperature in an acidic aqueous medium, which contains an aromatic aldehyde, which has a hydroxy group in the ortho-position to the aldehyde group.

2. A process according to claim 1, characterised in that the medium contains salicylic aldehyde.

3. A process according to claim 1 or 2, characterised in that the medium contains at least 0.01 mol of the aldehyde per mol of the optically-active S-(carboxymethyl)-cysteine.

4. A process according to any one of claims 1 to 3, characterised in that the medium has a pH of at most 1.

5. A process according to any one of claims 1 to 4, characterised in that the treatment takes place at a temperature of from 90 to 130°C.

## Revendications

1. Procédé pour la racémisation de S-(carboxyméthyl)-cystéine optiquement active, caractérisé en ce que la S-(carboxyméthyl)-cystéine optiquement active est traitée, à température élevée, dans un milieu aqueux acide contenant un aldéhyde aromatique qui comporte un groupe hydroxy en position ortho par rapport au groupe aldéhyde.

2. Procédé suivant la revendication 1, caractérisé en ce que le milieu contient de l'aldéhyde salicylique.

3. Procédé suivant l'une des revendications 1 ou 2, caractérisé en ce que le milieu contient au moins 0,01 mol d'aldéhyde par mol de S-(carboxyméthyl)-cystéine optiquement active.

4. Procédé suivant l'une des revendications 1 à 3, caractérisé en ce que le pH du milieu est de 1 au maximum.

5. Procédé suivant l'une quelconque des revendications 1 à 4, caractérisé en ce que le traitement est effectué à des températures se situant entre 90 et 130°C.